# EUROPEAN PATENT APPLICATION

(11) **EP 0 531 627 A1**
(43) Date of publication of application: **17.03.1993**
(21) Application number: 92109426.4
(22) Date of filing: 04.06.1992
(51) Int. Cl.: A61F 2/34

(54) **Endoprosthesis for cancer damaged hip bones**

(30) Priority: 10.09.1991 DE 9111221 U
(71) Applicant: Howmedica GmbH, D-24232 Schönkirchen (DE)
(72) Inventor: Robioneck, Bernd, Dipl.-Ing. B. Sc., W-2308 Schellhorn (DE)
(74) Representative: Dipl.-Ing. H. Hauck, Dipl.-Ing. E. Graalfs, Dipl.-Ing. W. Wehnert, Dr.-Ing. W. Döring, Dr.-Ing. N. Siemons

(57) **Abstract**

An endoprosthesis (10) for cancer damaged hip bones including a recess (50) in a distal portion (14) to receive a prosthetic hip socket whereby the endoprosthesis comprises an individual distal part (14) and an individual proximate part (12) which are secured to each other by means of a screwing connection (48), and that the distal and the proximal part include mounting brackets (56,58,20,26) for screwing said parts to the hip bone or, respectively, to a vertebra.

## Description

The invention refers to an endoprosthesis for cancer damaged hip bones according to the preamble of claim 1.

A variety of endoprostheses is known to replace the damaged joint ball of the femur and/or the joint socket of the hip bone when these parts do not anymore properly function. In cancer damaged hip bones, a large portion of the bony structure of the hip bone is attacted in many cases and is thus incapable to support a prosthetic hip socket in the usual manner. In such cases a unique bone substitute must be formed including a recess in the distal portion to receive a prosthetic hip socket. The individual forming of endoprostheses of this type is costly and takes too long a time.

It is known to first form a model of the bone portion to be substituted using X rays, in particular computer tomography, and then to form the endoprosthesis from the model.

The object of the present invention is to provide an endoprosthesis for cancer damaged hip bones which can be used in a wide variety Of individual shapes of the hip bone.

The object referred to is solved by an endoprosthesis according to claim 1.

The present invention is based on the knowledge that it is inevitable to have a photographic survey when cancer damaged parts lie within the region Of the natural hip socket or close thereto. It is further important that the prosthetic recess for receiving the prosthetic hip socket finds sufficient support and is safely secured to the hip bone.

The endoprosthesis according to the invention provides for a unique structure, but allows an individual adjustment with respect to the dimensions of the hip bone. According to the invention, the endoprosthesis comprises a distal and a proximal part which can be rigidly secured together by screws. The distal as well as the proximal part include mounting extensions to be secured by screws to the hip bone or, respectively, to a vertebra. Preferably, the proximal part is secured to the fifth vertebra and to the satrum.

The two-part structure of the endoprosthesis according to the invention facilitates making it in a die-casting process. Furthermore, the surgical fixing of the prosthesis parts is simplified due to the bipartite structure. For example, after secting certainbone portions of the pelvis, the proximal part is first fixed and then the distal part is secured to the proximal part, whereupon the proximal part is screwed to the vertebra.

For a safe connection between the distal and the proximal part, the invention provides for an interlaced connection. According to a further embodiment of the invention, both interlaced portions include interengaging faces, such as a nose, formed on the first part engaging a corresponding recess of the second part. The noses prevent a relative rotation of the parts even then when a single screw only is used for connecting both parts. Preferably, a pair of screws is used and the heads thereof are preferably embedded.

According to a still further embodiment of the invention, the distal part of the prosthesis is formed to comprise a pair of supporting portions of which one engages a pubic bone branch and the other an ischium branch, each supporting portion including a securing bracket. The supporting portions thus define bridge portions between the pubic bone branch or, respectively, ischium branch left and the portion of the distal prosthesis part in which the recess is formed to receive the prosthesis hip socket.

According to a still further embodiment, the proximal part includes a pair of securing brackets of which one having a BCS surface laterally engages the sacrum, and the other engages the vertebra in front leaving still a nerve passage. Each bracket preferably has a number of screw bores for receiving spongiosa screws to be secured to the associated vertebra.

A preferred embodiment of the present invention is described in more detail with reference to the drawings, which show:
- Fig. 1: a perspective view of an endoprosthesis according to the invention;
- Fig. 2: a perspective view of a part of a hip bone from the front including an endoprosthesis mounted thereon;
- Fig. 3: a view of the hip bone from the rear and a prosthesis mounted;
- Fig. 4: a left-hand view of a hip bone and a prosthesis mounted.

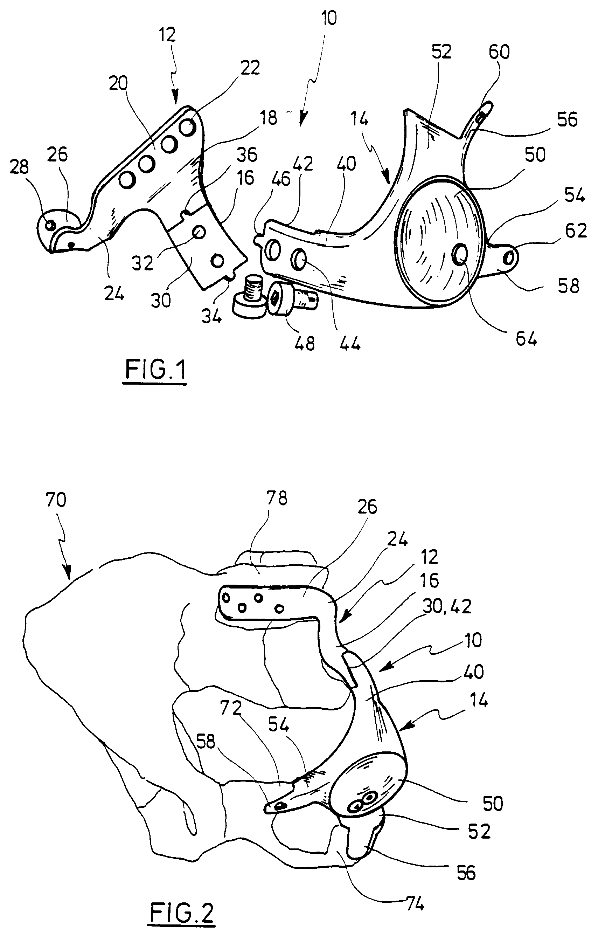

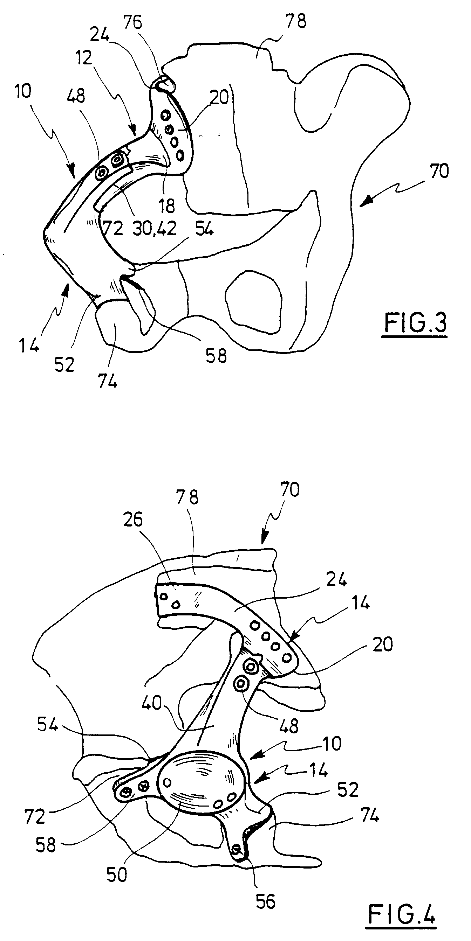

The endoprosthesis 10 illustrated in Figs. 1 to 4 comprises a proximal part 12 and a distal part 14. Both parts 12 and 14 are die-cast and are made of a body-compatible material. The mold is made in a known manner using computer tomography, for example, to survey the bone region to be substituted and making the mold according to the data obtained this way.

The distal part includes a plate-shaped portion merging via a bent-off portion 18 in a flat mounting bracket 20 including four bores 22 to receive bone screws not shown. The bracket extension 20 extends under a small angle with respect to the plane of the plate portion 16. The mounting portion 20 extends beyond a bent portion 24 to a second curved mounting bracket 26 including four bores 28 alike to receive bone screws not shown. The plate portion 16 includes an extension 30 in which two bores 32 are provided. A rounded nose 34 is provided at the free end of the plate portion 16. A recess 36 which is complementary with respect to the nose 34 is formed at a shoulder of the extension 30.

The distal part 14 comprises a plate portion 40 including an extension 42 including two bores 44. The extensions 30, 42 are complemantarily shaped, and the nose 46 of the portion 40 engages the recess 36 when the extensions 30, 42 are mounted in overlapping relationship. Screws 48 are used to secure the plate portions 16, 40 together, wherein the screw heads are received in the bores 44.

A recess 50 to receive a prosthetic hip socket made of a suitable plastic material for example is provided in the center region of the distal part 14. A first supporting portion 52 and a second supporting portion 54 including a lateral mounting bracket 56 and 58 each having a bore 60 to receive a bone screw, are formed on the side of the recess 50 opposite the plate portion 40. The supporting portions 52, 54 are provided with obtuse ends and surfaces having a microspherical structure for facilitating fixing to the bone. A number of bores is provided. The supporting portion 54 includes a bore which end 64 opens into the recess 50. The supporting portion 52 is provided with two bores as shown in Fig. 2. The bores receive screws for mounting the distal part to proper bone sections of the pelvis.

According to Fig. 2 a pelvis bone 70 is shown in a front view; the pubic bone and the ischium are resected on the left-hand side. Thus a pubic bone branch 72 and an ischium branch 74 are left. The surgical cut is selected such that the obtuse end of the supporting portions 52, 54 obtusely contacts the branches 72, 74. The mounting takes place through the mounting brackets 58 and 56. Furthermore, the supporting portions are secured by screws which are inserted through the bores opening into the recess 50.

In performing the surgical operation, the proximal part 14 is first mounted in the manner described and then the part 14 is screwed to the distal part 12 by using the screws 48. The mounting bracket 20 thus laterally engages the correlated vertebra (5th lumbral vertebra) which has to be shaped correspon-dingly. The mounting bracket is then secured to the vertebra by means of screws. The other mounting bracket 26 engages the front side of the vertebra and is mounted alike by means of screws, as particularly shown in Figs 2 and 4. The arched portion 24 leaves space for a nerve channel 76.

## Claims

1. An endoprosthesis for cancer damaged hip bones including a recess in a distal portion to receive a prosthetic hip socket, characterized in that the endoprosthesis comprises an individual distal part (14) and an individual proximate part (12) which are secured to each other by means of a screwing connection (48), and that the distal and the proximal part include mounting brackets (56, 58, 20, 25) for screwing said parts to the hip bone (70) or, respectively, to a vertebra (78).

2. The endoprosthesis of claim 1 characterized in that the proximal and distal part (12, 14) are shaped to positively interengage each other.

3. The endoprosthesis of claim 2, characterized in that the interengaging portions include complementary members (30, 42) including a nose (34, 46) each or the like which engages a complementary recess (36) in the other part.

4. The endoprosthesis of one of claims 1 to 3, characterized in that the distal part (14) includes a pair of supporting portions (52, 54) the first one being supported on a pubic bone branch (72) and the other being supported on an ischum branch (74), and that each supporting portion includes a mounting bracket (56, 58).

5. The endoprosthesis of one of claims 1 to 4, characterized in that the proximal part (12) includes two mounting brackets (20, 26), of which the first engages a vertebra (78) laterally and the other the front of the vertebra, wherein a nerve passage 76 is left.

6. The endoprosthesis of claim 5, characterized in that the mounting bracket (20) laterally engaging the vertebra (78) is formed to be flat, and the other mounting bracket is arched shaped.

7. The endoprosthesis of one of claims 1 to 6, characterized in that the ends of the supporting portions (52, 54) of the distal part (14) are shaped obtuse and preferably include a multiple spherical surface.

8. The endoprosthesis of one of claims 4 to 7 characterized in that the supporting portions (52, 54) include at least a through bore opening into the recess (50) to receive a screw each.
